(19) Europäisches Patentamt / European Patent Office / Office européen des brevets

(11) **EP 3 589 952 B1**

(12) # EUROPEAN PATENT SPECIFICATION

(45) Date of publication and mention of the grant of the patent: **23.09.2026 Bulletin 2026/39**

(21) Application number: **18760507.6**

(22) Date of filing: **27.02.2018**

(51) International Patent Classification (IPC):
***G01N 33/575** (2026.01)* ***G01N 33/50** (2006.01)*
***C12N 15/13** (2006.01)* ***A61K 51/10** (2006.01)*
***A61K 39/44** (2006.01)* ***B03C 1/005** (2006.01)*

(52) Cooperative Patent Classification (CPC):
**C07K 16/2896; A61K 31/198; A61K 31/454; A61K 31/475; A61K 31/675; A61K 31/704; C07K 16/2803; C07K 16/2878; C07K 16/289; G01N 33/54326; G01N 33/575;** B03C 1/01; B03C 2201/18; B03C 2201/26; G01N 2333/70596;
(Cont.)

(86) International application number: **PCT/US2018/019925**

(87) International publication number: **WO 2018/160553 (07.09.2018 Gazette 2018/36)**

(54) **IMPROVED KITS AND ASSAYS TO DETECT CIRCULATING MULTIPLE MYELOMA CELLS FROM BLOOD**

VERBESSERTE KITS UND TESTS ZUM NACHWEIS ZIRKULIERENDER MULTIPLER MYELOMZELLEN AUS BLUT

KITS ET DOSAGES AMÉLIORÉS POUR DÉTECTER DES CELLULES DE MYÉLOME MULTIPLE CIRCULANTES À PARTIR DU SANG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.02.2017 US 201762464585 P**

(43) Date of publication of application: **08.01.2020 Bulletin 2020/02**

(60) Divisional application: **26194598.4**

(73) Proprietor: **Menarini Silicon Biosystems S.p.A.**
**40013 Castel Maggiore (BO) (IT)**

(72) Inventors:
- **FOULK, Brad**
  **Chalfont, Pennsylvania 18914 (US)**
- **RAO, Galla Chandra**
  **Princeton Jct., New Jersey 08550 (US)**
- **GROSS, Steven**
  **Ambler, Pennsylvania 19002 (US)**
- **MORANO, Carrie Capps**
  **Hatfield, Pennsylvania 19440 (US)**

(74) Representative: **Hoffmann Eitle**
**Patent- und Rechtsanwälte PartmbB**
**Arabellastraße 30**
**81925 München (DE)**

(56) References cited:
**WO-A1-2014/124280 US-A1- 2013 189 675**

- **STEVEN GROSS ET AL: "Automated Enumeration and Characterization of Circulating Multiple Myeloma Cells in Blood", BLOOD 2011 118:1825, 1 January 2011 (2011-01-01), XP055564281, Retrieved from the Internet <URL:http://www.bloodjournal.org/content/118/21/1825> [retrieved on 20190304]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **A. J. NOVAK: "Expression of BCMA, TACI, and BAFF-R in multiple myeloma: a mechanism for growth and survival", BLOOD, vol. 103, no. 2, 15 January 2004 (2004-01-15), pages 689 - 694, XP055004448, ISSN: 0006-4971, DOI: 10.1182/ blood-2003-06-2043**
- **J CHU ET AL: "CS1-specific chimeric antigen receptor (CAR)-engineered natural killer cells enhance in vitro and in vivo antitumor activity against human multiple myeloma", LEUKEMIA, vol. 28, no. 4, 26 September 2013 (2013-09-26), pages 917 - 927, XP055133640, ISSN: 0887-6924, DOI: 10.1038/leu.2013.279**
- **LYMPHOID NEOPLASIA ET AL: "Brief Report Robust isolation of malignant plasma cells in multiple myeloma", BLOOD, vol. 123, no. 9, 27 February 2014 (2014-02-27), pages 1336 - 1340, XP055762069, DOI: 10.1182/ blood-2013-09-529800**
- **ANDREE KIKI C ET AL: "Challenges in circulating tumor cell detection by the CellSearch system", MOLECULAR ONCOLOGY, ELSEVIER, vol. 10, no. 3, 25 December 2015 (2015-12-25), pages 395 - 407, XP029432259, ISSN: 1574-7891, DOI: 10.1016/ J.MOLONC.2015.12.002**
- **J.F. SWENNENHUIS ET AL: "Improving the CellSearch? system", EXPERT REVIEWS IN MOLECULAR DIAGNOSTICS, vol. 16, no. 12, 17 November 2016 (2016-11-17), GB, pages 1291 - 1305, XP055415768, ISSN: 1473-7159, DOI: 10.1080/14737159.2016.1255144**
- **BRAD FOULK ET AL: "Abstract 3163: Peripheral blood circulating multiple myeloma cells (CMMCs) correlate with disease burden and can be used to characterize high-risk cytogenetics in newly diagnosed and smoldering myeloma", CLINICAL RESEARCH (EXCLUDING CLINICAL TRIALS), 15 July 2016 (2016-07-15), pages 3163 - 3163, XP055762327, DOI: 10.1158/ 1538-7445.AM2016-3163**
- **BRAD FOULK ET AL: "Enumeration and characterization of circulating multiple myeloma cells in patients with plasma cell disorders", BRITISH JOURNAL OF HAEMATOLOGY, vol. 180, no. 1, 5 November 2017 (2017-11-05), GB, pages 71 - 81, XP055762090, ISSN: 0007-1048, DOI: 10.1111/bjh.15003**
- **HSI, ED ET AL.: "CS1, a Potential New Therapeutic Antibody Target for the Treatment Of Multiple Myeloma", CLINICAL CANCER RESEARCH, vol. 14, no. 9, 1 May 2008 (2008-05-01), pages 2775 - 2784, XP002571730**
- **LEE, L ET AL.: "Evaluation of B cell maturation antigen as a target for antibody drug conjugate mediated cytotoxicity in multiple myeloma", BRITISH JOURNAL OF HAEMATOLOGY, vol. 174, no. 6, September 2016 (2016-09-01), pages 911 - 922, XP055331908**
- **TAI, YT ET AL.: "Anti- CS 1 humanized monoclonal antibody HuLuc63 inhibits myeloma cell adhesion and induces antibody-dependent cellular cytotoxicity in the bone marrow milieu", BLOOD, vol. 112, no. 4, 15 August 2008 (2008-08-15), pages 1329 - 1337, XP002571731**


(52) Cooperative Patent Classification (CPC): (Cont.)
G01N 2800/52

## Description

BACKGROUND

Multiple Myeloma (also known as myeloma or plasma cell myeloma) is a progressive hematologic cancer of the plasma cell. The condition is characterized by excessive numbers of plasma cells in the bone marrow and overproduction of intact monoclonal immunoglobulin or free monoclonal light chains.

Clinically the disease is diagnosed, staged, and treated based on a variety of parameters which include the myeloma tumor cell mass on the basis of the amount of monoclonal (or myeloma) protein (M protein) in the serum and/or urine, along with hemoglobin and serum calcium concentrations, the number of lytic bone lesions based on a skeletal survey, and the presence or absence of renal failure. Additional approaches to characterizing the condition include the detection of greater than ten percent (10%) of plasma cells on a bone marrow examination, the presence of soft tissue plasmacytomas and the detection of free kappa and lambda serum immunoglobulin light chain. Bone marrow examination is done using standard histology and immunohistochemistry techniques. Additional cytogenetics of bone marrow samples may be conducted to determine prognosis. Follow up surveillance consists of chemistry and bone marrow evaluations if clinically indicated due to its invasive nature.

Andree et al., Molecular Oncology, 2016, 10: 395-407, Challenges in circulating tumor cell detection by the CellSearch system, provide a review on enumeration and characterization of circulating tumor cells.

Swennenhuis et al., Expert Review of Molecular Diagnostics, 2016, 16(12): 1291-1305, Improving the CellSearch system, provide a review on work performed with the CellSearch system.

SUMMARY OF THE INVENTION

The invention is as set out in the claims. In the present invention, the rare cells are circulating multiple myeloma cells (CMMCs). The invention described herein includes a reagent and methods for capturing, detecting, enumerating or analyzing rare cells from blood samples using a reagent comprising colloidal magnetic particles comprising a ligand that specifically binds to CD 138 and a ligand that specifically binds to a protein selected from group consisting of CS1 and BCMA.

In some embodiments, disclosed herein is a reagent for capturing rare cells from biological samples, comprising colloidal magnetic particles, wherein said magnetic particles are conjugated to: (i) a first ligand that specifically binds to CD 138; and (ii) a second ligand selected from group consisting of a ligand that specifically binds to CD2 subset-1 protein (CS1) and a ligand that specifically binds to B-cell maturation antigen (BCMA). In some embodiments, the first ligand is selected from an antibody, an antibody fragment, a protein, a polypeptide, an enzyme, and an aptamer. In some embodiments, the second ligand is selected from an antibody, an antibody fragment, a protein, a polypeptide, an enzyme, and an aptamer. In some embodiments, the first ligand is an antibody, or fragment thereof. In some embodiments, the second ligand is an antibody, or fragment thereof. In the present invention, the rare cells are circulating multiple myeloma cells (CMMCs). In some embodiments, the second ligand specifically binds to BCMA. In some embodiments, the second ligand specifically binds to CS1. In some embodiments, said magnetic particles comprise a ligand that specifically binds to CS1 and a ligand that specifically binds to BCMA.

In some embodiments, disclosed herein is a composition comprising a reagent, as disclosed herein, and a stabilizing agent, wherein said stabilizing agent is a dialdehyde. In some embodiments, the dialdehyde is selected from the group selected from the group consisting of glutaraldehyde, glyoxal, and combinations thereof. In some embodiments, the dialdehyde is glyoxal. In some embodiments, the composition comprises CellSave® liquid. In some embodiments, the composition comprises CellSecure® liquid. In some embodiments, the stabilizing agent is present in an amount of about 0.1 to about 50% w/v. In some embodiments, the stabilizing agent is present in an amount of about 0.3 to about 30% w/v. In some embodiments, the stabilizing agent is present in an amount of about 0.3 to about 5% w/v.

Disclosed herein, in some embodiments, is a kit for capturing rare cells from biological samples comprising a) a reagent as disclosed herein or a composition as disclosed herein and b) at least one additional marker. In some embodiments, the additional marker is selected from the group consisting of DAPI, and a ligand that specifically binds to a protein selected from: CS1, BCMA, CD19, CD45, CD 56, immunoglobulin lambda, immunoglobulin kappa, CD 200, and Ki67. In some embodiments, the kit further comprises a second additional marker and a third additional marker. In some embodiments, the at least one additional marker is a ligand that specifically binds to CS1, the second additional marker is DAPI and the third additional marker is a ligand that specifically binds to CD45. In some embodiments, the kit further comprises three additional markers, wherein at least one additional marker is a ligand that specifically binds to CS1, and the three additional markers are DAPI, a ligand that specifically binds to CD19, and a ligand that specifically binds to CD45. In some embodiments, the kit further comprises four additional markers, wherein at least one additional marker is a ligand that specifically binds to CS1, and the four additional markers are a ligand that specifically binds to BCMA, DAPI, a ligand that specifically binds to CD19, and a ligand that specifically binds to CD45. In some embodiments, all additional

markers that are not DAPI are antibodies or fragments thereof.

Disclosed herein, in some embodiments, is a method for capturing rare cells from a biological sample obtained from a patient, comprising a) contacting the biological sample with a reagent disclosed herein or a composition disclosed herein; and b) subjecting the sample of step (a) to a magnetic field to produce a separated fraction of magnetic particle-bound rare cells. In some embodiments, the method further comprises contacting the biological sample with at least one additional marker. In the present invention, the rare cells are CMMCs. In some embodiments, the at least one additional marker is selected from the group consisting of DAPI, and a ligand that specifically binds to a protein selected from: CS1, BCMA, CD19, CD45, CD 56, immunoglobulin lambda, immunoglobulin kappa, CD 200, and Ki67. In some embodiments, the method further comprises treating the sample with a second additional marker and a third additional marker. In some embodiments, the at least one additional marker is a ligand that specifically binds to CS1, the second additional marker is DAPI and the third additional marker is a ligand that specifically binds to CD45. In some embodiments, the method further comprises treating the sample with three additional markers, wherein at least one additional marker is a ligand that specifically binds to CS1, and the three additional markers are DAPI, a ligand that specifically binds to CD19, and a ligand that specifically binds to CD45. In some embodiments, the method further comprises treating the sample with four additional markers, wherein at least one additional marker is a ligand that specifically binds to CS1, and the four additional markers are a ligand that specifically binds to BCMA, DAPI, a ligand that specifically binds to CD19, and a ligand that specifically binds to CD45. In some embodiments, the sample has a volume of about 2 mL to about 10 mL. In some embodiments, the sample has a volume of about 3 mL to about 7.5 mL. In some embodiments, the sample has a volume of about 4 mL. In some embodiments, the sample has a volume of about 7.5 mL. In some embodiments, the methods disclosed herein comprise contacting the biological sample with a composition disclosed herein.

In some embodiments, disclosed herein is a method of detecting the amount of rare cells in a biological sample from a patient, comprising (a) contacting a biological sample obtained from a patient with a reagent disclosed herein or a composition disclosed herein; (b) subjecting the sample of step (a) to a magnetic field to produce a separated fraction of magnetic particle-bound rare cells; and (c) detecting the number of magnetic particle-bound rare cells. In some embodiments, the method further comprises treating the sample of step (a) with at least one additional marker. In the present invention, the rare cells are CMMCs. In some embodiments, the at least one additional marker is selected from the group consisting of DAPI, and a ligand that specifically binds to a protein selected from: CS1, BCMA, CD19, CD45, CD 56, immunoglobulin lambda, immunoglobulin kappa, CD 200, and Ki67. In some embodiments, the method further comprises treating the sample with a second additional marker and a third additional marker. In some embodiments, the at least one additional marker is a ligand that specifically binds to CS1, the second additional marker is DAPI and the third additional marker is a ligand that specifically binds to CD45. In some embodiments, the method further comprises treating the sample with three additional markers, wherein at least one additional marker is a ligand that specifically binds to CS1, and the three additional markers are DAPI, a ligand that specifically binds to CD19, and a ligand that specifically binds to CD45. In some embodiments, the method further comprises treating the sample with four additional markers, wherein at least one additional marker is a ligand that specifically binds to CS1, and the four additional markers are a ligand that specifically binds to BCMA, DAPI, a ligand that specifically binds to CD19, and a ligand that specifically binds to CD45. In some embodiments, the sample has a volume of about 2 mL to about 10 mL. In some embodiments, the sample has a volume of about 3 mL to about 7.5 mL. In some embodiments, the sample has a volume of about 4 mL. In some embodiments, the sample has a volume of about 7.5 mL. In some embodiments, the methods disclosed herein comprise contacting the biological sample with a composition disclosed herein.

Disclosed herein, in some embodiments, is a method of determining if a patient is a likely candidate for therapeutic intervention for a disease associated with abnormal plasma cells, comprising (a) detecting the amount of rare cells in a biological sample from a patient according to a method disclosed herein; and (b) determining if the number of rare cells present in said sample, is equal to or greater than or equal to a normal range, wherein an amount of rare cells present in said sample greater than a normal range indicate the patient is a likely candidate for therapeutic intervention for a disease associated with abnormal plasma cells. In the present invention, the rare cells are CMMCs. In some embodiments, the normal range of CMMCs in a patient sample is less than 7 CMMCs in about 2 mL to 10 mL of blood. In some embodiments, the disease associated with abnormal plasma cells is selected from multiple myeloma, monoclonal gammopathy of undetermined significance (MGUS), and smoldering multiple myeloma. In some embodiments, the disease associated with abnormal plasma cells is multiple myeloma.

Disclosed herein, in some embodiments, is a method of determining whether a patient undergoing therapeutic intervention for a disease associated with abnormal plasma cells is being effectively treated, comprising (a) detecting the amount of rare cells in a biological sample from a patient according to a method disclosed herein at a first point in time; and (b)detecting the amount of rare cells in a biological sample from the patient according to a method disclosed herein at a second subsequent point in time; and (c) comparing the numbers of rare cells in a biological sample from the patient between the first point in time and the second subsequent point in time. In some embodiments, a lesser amount of rare cells in the biological sample from patient at the second subsequent point in time indicates that the patient is being effectively treated. In the present invention, the rare cells are CMMCs. In some embodiments, the disease associated with abnormal

plasma cells is selected from multiple myeloma, monoclonal gammopathy of undetermined significance (MGUS), and smoldering multiple myeloma. In some embodiments, the disease associated with abnormal plasma cells is multiple myeloma. In some embodiments, said therapeutic intervention is the administration of one of the following drugs daratumumab, isatuximab, examethasone, cyclophosphamide, Vincristine, Bortezomib, Melphalan, Zometa, Aloxi, Lenalidomide,or Doxirubicin.

BRIEF DESCRIPTION OF FIGURE

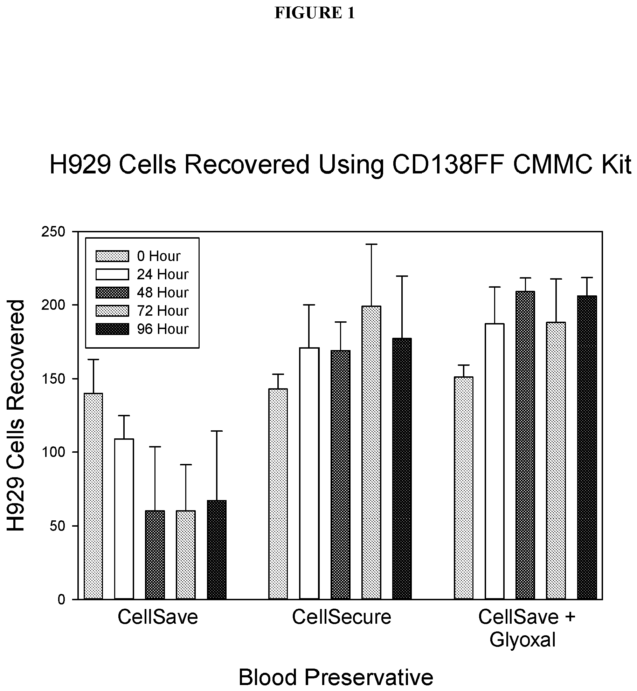

**Figure** 1 illustrates the number of H929 cells recovered using CD138 ferrofluid in either CellSave® liquid alone, CellSecure® liquid, or CellSave® with added glyoxal. Numbers of H929 cells were detected at 0, 24, 48, 72 and 96 hours after addition of blood preservative.

DETAILED DESCRIPTION

The invention described herein includes reagents and compositions for capturing circulating multiple myeloma cells from biological samples.

Currently, flow cytometric analysis of bone marrow is used as a tool for disease characterization and to distinguish between neoplastic plasma cell disorders from normal plasma cells and to detect minimal residual disease. Nonetheless, this approach continues to rely on an invasive procedure. There is significant need to develop less invasive techniques to detect, monitor and characterize the disease and the presence of these cells in the blood provides that opportunity.

More sensitive tools need to be developed for more accurate assessment of risk and monitoring for progression of diseases of abnormal plasma cells in earlier stages of disease, including monoclonal gammopathy of undetermined significance (MGUS) and Smoldering Multiple Myeloma. Research data suggests that circulating multiple myeloma cells (CMMCs) can be detected in earlier stages of disease and may correlate with prognosis, supporting the use of a standardized methodology to capture, enumerate and characterize these cells in earlier stages of disease.

CMMCs are CD138 positive and commercial kits using CD138 magnetic particles are available to isolate CMMCs. Stem Cell Technologies has an EasySep® Human CD138 Positive Selection Kit which can select CD138 positive cells from bone marrow and peripheral blood mononuclear cells (PBMCs). Miltenyi Biotech has CD138 Microbeads for the selection of CD138 positive cells from bone marrow, PBMC and whole blood. Analysis of collected samples is typically performed using flow cytometry. However, these tests have their drawbacks. In some cases such as bone marrow, the methods are invasive, in other cases, the tests give variable results when searching for CMMCs. Ferrofluids that are conjugated to anti CD 138 and anti CD 38 have been used for a capturing CMMCs from patient samples. See U.S. Patent No. 9,618,515. This non-invasive test and this test has been successfully used to capture CMMCs. The following invention provides other compositions and methods for isolation enumeration and flexible molecular characterization of CMMCs from peripheral blood.

The term "rare cells" means a cell, a small cluster of cells, or a class of cells and their associated events that are not readily and reliably detected, or accurately quantified, in biological samples without some form of positive or negative selection enrichment or concentration being applied to the sample. In the present invention, rare cells are circulating multiple myeloma cells ("CMMCs").

The term "biological sample" means naturally occurring extracts of a patient. Examples of such extract include but are not limited to blood, bone marrow, urine and plasma. In some embodiments, the biological sample used in the methods disclosed herein is blood.

The term "ligand" refers to a molecule that specifically binds to another molecule. In some embodiments, a ligand is selected from an antibody, an antibody fragment, a protein, a polypeptide, an enzyme, and an aptamer. In some embodiments, the a ligand is an antibody, or fragment thereof.

Such ligands may be conjugated to colloidal magnetic particles by methods that are substantially similar to the methods disclosed U.S. Patent No. 6,365,362.

The term "colloidal magnetic particles" refers to particles that are metallic or organometallic. Examples of such particles are disclosed in U.S. Pat. Nos. 5,597,531; 5,698,271; and 6,365,362. Such particles may be optionally coated with a polymer, preferably a polymer of biological origin such as bovine serum albumin and casein. The preferred coating polymer is bovine serum albumin.

***Reagents***

In some embodiments, disclosed herein is a reagent for capturing rare cells from biological samples, comprising colloidal magnetic particles, wherein said magnetic particles are conjugated to: (i) a first ligand that specifically binds to CD 138; and (ii) a second ligand selected from group consisting of a ligand that specifically binds to CD2 subset-1 protein

(CS1) and a ligand that specifically binds to B-cell maturation antigen (BCMA). In the present invention, the rare cells are circulating multiple myeloma cells (CMMCs).

In some embodiments, the first ligand is selected from an antibody, an antibody fragment, a protein, a polypeptide, an enzyme, and an aptamer. In some embodiments, the second ligand is selected from an antibody, an antibody fragment, a protein, a polypeptide, an enzyme, and an aptamer. In some embodiments, the first ligand is an antibody, or fragment thereof. In some embodiments, the second ligand is an antibody, or fragment thereof.

In some embodiments, the second ligand specifically binds to BCMA. In some embodiments, the second ligand specifically binds to CS1. In some embodiments, said magnetic particles comprise a ligand that specifically binds to CS1 and a ligand that specifically binds to BCMA.

In some embodiments, either the magnetic particles, first ligand, second ligand, or a combination thereof are detectably labelled. In some embodiments, a detectable label is chosen from the group of consisting of a radiolabel, an enzymatic label, a chemiluminescent label, a fluorescent label and a colorimetric label.

*Compositions*

In some embodiments, disclosed herein is a composition comprising a reagent, as disclosed herein, and a stabilizing agent.

In some embodiments, said stabilizing agent is a dialdehyde. In some embodiments, the dialdehyde is selected from the group selected from the group consisting of glutaraldehyde, glyoxal, and combinations thereof. In some embodiments, the dialdehyde is glyoxal.

In some embodiments, the composition comprises CellSave® liquid, manufactured by Menarini Silicon Biosystems.

In some embodiments, the composition comprises CellSecure® liquid, manufactured by Menarini Silicon Biosystems, which itself comprises glyoxal.

In some embodiments, the stabilizing agent is present in an amount of about 0.1 to about 50% w/v. In some embodiments, the stabilizing agent is present in an amount of about 0.3 to about 30% w/v. In some embodiments, the stabilizing agent is present in an amount of about 0.3 to about 5% w/v. In some embodiments, the stabilizing agent is present in an amount between 0.1% and an amount selected from the group consisting of less than about 40%, less than about 30%, less than about 25%, less than about 20%, less than about 15%, less than about 10%, less than about 9%, less than about 8%, less than about 7%, less than about 6%, less than about 5%, less than about 4%, less than about 3%, less than about 2%, and less than about 1%. In some embodiments, the stabilizing agent is present in an amount between 1 % and an amount selected from the group consisting of less than about 40%, less than about 30%, less than about 25%, less than about 20%, less than about 15%, less than about 10%, less than about 9%, less than about 8%, less than about 7%, less than about 6%, less than about 5%, less than about 4%, less than about 3%, and less than about 2%. In some embodiments, the stabilizing agent is present in an amount between 5% and an amount selected from the group consisting of less than about 40%, less than about 30%, less than about 25%, less than about 20%, less than about 15%, less than about 10%, less than about 9%, less than about 8%, less than about 7%, and less than about 6%. In some embodiments, the stabilizing agent is present in an amount between 10% and an amount selected from the group consisting of less than about 40%, less than about 30%, less than about 25%, less than about 20%, and less than about 15%. In some embodiments, the stabilizing agent is glyoxal.

*Kits*

Disclosed herein, in some embodiments, is a kit for capturing rare cells from biological samples comprising a) a reagent as disclosed herein or a composition as disclosed herein and b) at least one additional marker.

In some embodiments, disclosed herein is a kit for capturing rare cells from biological samples comprising (a) colloidal magnetic particles conjugated to at least (i) anti-CD 138 and (ii) a ligand selected from group consisting of anti CS1 and anti BCMA, and (b) at least one additional marker.

As used herein, the term "additional marker" refers to a molecule that can be used to assist in discerning from one type of cell from another. In some embodiments an additional marker is a cell associated protein that is specific for CMMC or excludes CMMCs. Such proteins include but are not limited to antibodies selected from the group consisting of anti CS1, anti BCMA, anti CD19, anti CD45, anti CD 56, anti lambda, anti kappa, anti CD 200, and anti Ki67. Such antibodies may be detectably labeled, such as with fluorescent labels like phycoertythrin ("PE"), fluorescein isothiocyanate, and allophycocyanin ("APC"). In some embodiments, an additional marker is a nucleic acid dye, such as DAPI. In some embodiments, an additional marker is selected from the group consisting of anti CS1, anti CD19, anti CD45, anti Ki67 and DAPI. In some embodiments, the additional marker is selected from the group consisting, anti-CD45-APC (conjugated to APC) anti-CD19-APC, and DAPI. In some embodiments, at least two additional markers are used in component (b) more preferably, three additional markers, most preferably four additional markers.

In some embodiments, the additional marker is selected from the group consisting of DAPI, and a ligand that specifically binds to a protein selected from: CS1, BCMA, CD19, CD45, CD 56, immunoglobulin lambda, immunoglobulin kappa, CD 200, and Ki67. In some embodiments, the kit further comprises a second additional marker and a third additional marker. In some embodiments, the at least one additional marker is a ligand that specifically binds to CS1, the second additional marker is DAPI and the third additional marker is a ligand that specifically binds to CD45. In some embodiments, the kit further comprises three additional markers, wherein at least one additional marker is a ligand that specifically binds to CS1, and the three additional markers are DAPI, a ligand that specifically binds to CD19, and a ligand that specifically binds to CD45. In some embodiments, the kit further comprises four additional markers, wherein at least one additional marker is a ligand that specifically binds to CS1, and the four additional markers are a ligand that specifically binds to BCMA, DAPI, a ligand that specifically binds to CD19, and a ligand that specifically binds to CD45. In some embodiments, all additional markers that are not DAPI are antibodies or fragments thereof.

In some embodiments, the kit further comprises a stabilizing agent. In some embodiments, said stabilizing agent is a dialdehyde. In some embodiments, the dialdehyde is selected from the group selected from the group consisting of glutaraldehyde, glyoxal, and combinations thereof. In some embodiments, the dialdehyde is glyoxal.

In some embodiments, the kit further comprises CellSave® liquid, manufactured by Menarini Silicon Biosystems.

In some embodiments, the kit further comprises CellSecure® liquid, manufactured by Menarini Silicon Biosystems.

In some embodiments, disclosed herein is a method of capturing rare cells from a biological sample obtained from a patient, comprising a) contacting the biological sample with colloidal magnetic particles conjugated to a ligand that specifically binds to CD138 and a stabilizing agent, wherein the stabilizing agent is a dialdehyde; and b) subjecting the sample of step (a) to a magnetic field to produce a separated fraction of magnetic particle-bound rare cells. In some embodiments, the dialdehyde is selected from the group selected from the group consisting of glutaraldehyde, glyoxal, and combinations thereof. In some embodiments, the dialdehyde is glyoxal. In some embodiments, step a) occurs up to 24, 48, 96 hours or more after the biological sample is obtained from the patient.

***Methods of Use***

Disclosed herein, in some embodiments, is a method for capturing rare cells from a biological sample obtained from a patient, comprising a) contacting the biological sample with a reagent disclosed herein or a composition disclosed herein; and b) subjecting the sample of step (a) to a magnetic field to produce a separated fraction of magnetic particle-bound rare cells. In some embodiments, the method further comprises contacting the biological sample with at least one additional marker. In the present invention, the rare cells are CMMCs.

In some embodiments, disclosed herein is a method of detecting the amount (i.e., number) of rare cells in a biological sample from a patient, comprising (a) contacting a biological sample obtained from a patient with a reagent disclosed herein or a composition disclosed herein; (b) subjecting the sample of step (a) to a magnetic field to produce a separated fraction of magnetic particle-bound rare cells; and (c) detecting the number of magnetic particle-bound rare cells. In some embodiments, the method further comprises treating the sample of step (a) with at least one additional marker. In the present invention, the rare cells are CMMCs.

In some embodiments, in the methods disclosed herein, the at least one additional marker is selected from the group consisting of DAPI, and a ligand that specifically binds to a protein selected from: CS1, BCMA, CD19, CD45, CD 56, immunoglobulin lambda, immunoglobulin kappa, CD 200, and Ki67. In some embodiments, the method further comprises treating the sample with a second additional marker and a third additional marker. In some embodiments, the at least one additional marker is a ligand that specifically binds to CS1, the second additional marker is DAPI and the third additional marker is a ligand that specifically binds to CD45. In some embodiments, the method further comprises treating the sample with three additional markers, wherein at least one additional marker is a ligand that specifically binds to CS1, and the three additional markers are DAPI, a ligand that specifically binds to CD19, and a ligand that specifically binds to CD45. In some embodiments, the method further comprises treating the sample with four additional markers, wherein at least one additional marker is a ligand that specifically binds to CS1, and the four additional markers are a ligand that specifically binds to BCMA, DAPI, a ligand that specifically binds to CD19, and a ligand that specifically binds to CD45.

The "magnetic field" may be produced by any of a number of methods, particularly by two magnetic separators substantially as described in U.S. Pat. No 7,901,950.

In some embodiments, detecting the number of magnetic particle-bound rare cells is conducted visually or electronically. In some embodiments, the degree of fluorescence of a magnetically captured sample is measured. Such analysis methods are disclosed in U.S. Pat. No. 7,011,794.

In some embodiments, in the methods disclosed herein, the sample has a volume of about 2 mL to about 10 mL. In some embodiments, the sample has a volume of about 3 mL to about 7.5 mL. In some embodiments, the sample has a volume of about 4 mL. In some embodiments, the sample has a volume of about 7.5 mL.

In some embodiments, the methods disclosed herein comprise contacting the biological sample with a

composition disclosed herein.

In some embodiments, the methods of capturing and detecting rare cells in a biological sample disclosed herein can further be used to make various determinations in regards to disease of abnormal plasma cells. In some embodiments, the disease of abnormal plasma cells is a plasma cell neoplasm. In some embodiments, the disease or abnormal plasma cells is plasmacytoma. In some embodiments, the disease associated with abnormal plasma cells is selected from multiple myeloma, monoclonal gammopathy of undetermined significance (MGUS), and smoldering multiple myeloma. In some embodiments, the disease associated with abnormal plasma cells is multiple myeloma.

As such, disclosed herein, in some embodiments, is a method of determining if a patient is a likely candidate for therapeutic intervention for a disease associated with abnormal plasma cells, comprising (a) detecting the amount of rare cells in a biological sample from a patient according to a method disclosed herein; and (b) determining if the number of rare cells present in said sample, is equal to or greater than or equal to a normal range, wherein an amount of rare cells present in said sample greater than a normal range indicate the patient is a likely candidate for therapeutic intervention for a disease associated with abnormal plasma cells. In the present invention, the rare cells are CMMCs.

The term "normal range" refers to the average number of CMMC cells present in a blood sample from a population that does not have diseases associated with abnormal plasma cells. In some embodiments, the normal range of CMMCs in a patient sample is less than 7 CMMCs in about 2 mL to 10 mL of blood. The higher this number, the more likely it is that the patient either has one of the diseases associated with abnormal plasma cells. If a patient has between 8 and 20 CMMCs in a sample of blood such patient has a higher probability of having one of the diseases associated with abnormal plasma cells. If a patient has between 21 and 49 CMMCs the patient has an elevated level and is more likely to have one of the diseases associated with abnormal plasma cells, if a patient has between 50 and tens of thousands of CMMCs that patient has a highly elevated level and even more likely to have one of such diseases.

In some embodiments, the disease associated with abnormal plasma cells is selected from multiple myeloma, monoclonal gammopathy of undetermined significance (MGUS), and smoldering multiple myeloma. In some embodiments, the disease associated with abnormal plasma cells is multiple myeloma.

Disclosed herein, in some embodiments, is a method of determining whether a patient undergoing therapeutic intervention for a disease associated with abnormal plasma cells is being effectively treated, comprising (a) detecting the amount of rare cells in a biological sample from a patient according to a method disclosed herein at a first point in time; and (b)detecting the amount of rare cells in a biological sample from the patient according to a method disclosed herein at a second subsequent point in time; and (c) comparing the numbers of rare cells in a biological sample from the patient between the first point in time and the second subsequent point in time. In some embodiments, a lesser amount of rare cells in the biological sample from patient at the second subsequent point in time indicates that the patient is being effectively treated. In the present invention, the rare cells are CMMCs.

Such therapeutic intervention includes but is not limited to visiting a physician, obtaining therapeutic treatment such as radiation, and treatment with of drugs that treat any of the diseases associated with abnormal plasma levels, and monitoring the effect of such therapeutic treatments. For example, if a patient is being treated with a drug, the patient's levels of CMMC may be assessed during the course of treatment to determine if the drug is working. Such drugs include but are not limited to daratumumab, isatuximab, examethasone, cyclophosphamide, vincristine, bortezomib, melphalan, zometa, palonosetron, lenalidomide, doxirubicin, and the like. The preferred drugs are daratumumab and isatuximab, most preferably daratumumab. Patients who take the drugs that modulate CD38, such as daratumumab and istuximab, particularly benefit from the kits and methods of this invention. If one uses a capture agent that includes anti CD38, tests of the same samples yield variable results and not all rare cells are captured. Further, patients that receive therapeutics that target CD38, such as daratumumab, will benefit from this invention. The presence of CD38 binding therapeutics in a patient's blood can interfere with the binding of CMMC capture or detection reagent and result in fewer CNMMCs being captured or detected.

In some embodiments, said therapeutic intervention is selected from surgery, radiation therapy, and chemotherapy.

In some embodiments, said therapeutic intervention is the administration of one of the following drugs daratumumab, isatuximab, examethasone, cyclophosphamide, Vincristine, Bortezomib, Melphalan, Zometa, Aloxi, Lenalidomide,or Doxirubicin.

EXAMPLES

**Example 1 Capture** targets

Circulating Multiple Myeloma Cells (CMMC), a form of abnormal plasma cells, captured from blood have been captured and analyzed using the CellTracks® AutoPrep® and CellTracks Analyzer II® System. In this procedure, a combination of colloidal magnetic particles comprising anti-CD 138 and a ligand selected from group consisting of anti CS1 and anti BCMA as a capture agent and dyes (such as the nucleic acid dye DAPI) are used to identify abnormal plasma cells

and to distinguish them from contaminating leukocytes and debris. For this reason anti-CD138 and another of the identified ligands was coupled to ferrofluid, magnetic nanoparticles, which are used to magnetically select CMMCs from a sample of peripheral blood. In order to detect the abnormal cells from contaminating leukocytes several fluorescent biomarkers are used. Ant-CS1 is conjugated to phycoerythrin (PE) and is used as a positive marker for the detection of plasma cells. The method also uses allophycocyanine (APC) conjugated anti- CD45 and anti-CD19 conjugated to allophycocyanin (APC) as a negative marker. CD45 is a pan-leukocyte marker found on peripheral blood leukocytes and CD19 is a specific B cell marker. Myeloma cells are functionally differentiated B cells which do not express either CD45 or CD19. A final marker in this assay is anti-CD56 conjugated to fluorescein isothiocyanate (FITC). CD56 can be found on some peripheral leukocyte subsets such as NK cells but is also expressed on 75% of myeloma cells and is often associated with poorer patient prognosis. So while CD56 is neither a positive or negative marker for multiple myeloma its expression levels on cells can be monitored during patient drug therapy.

The enriched and stained cells were transferred to a CellTracks® cartridge and MagNest® for magnetic mounting. The cartridge was scanned using the Cell Tracks Analyzer II®. Individual images of cells were presented to the operator for review, and scored as CMMCs, based on fluorescence and cell morphology.

Abbreviations

PE-Phycoerythrin

FITC-Fluorescein isothiocyanate

APC-Allophycocyanin

PBMC-peripheral blood mononuclear cell

Antibody Sources-

CD138:

Gen-Probe Diaclone SAS

1 Bd A Fleming, BP 1985

F-25020 Besancon Cedex, France

CD38 CD19

R&D Systems

614 McKinley Place N.E.

Minneapolis, MN 55413

Antibodies to different markers present on myeloma cells were conjugated to colloidal magnetic ferrofluid (FF) particles and were used as capture reagents to capture myeloma cells from blood. One of the markers is a cell surface glycoprotein ("CS1" a.k.a. CD319) which is highly expressed on myeloma cells. The other marker is B cell maturation antigen ("BCMA" a.k.a. CD269) which is expressed on normal and malignant plasma cells. Magnetic particles were developed that were coupled to both anti- CD138 and anti-CS1 or anti-CD138 and anti-BCMA. Using the methods described for creating anti CD 138 and anti CD 38 ferrofluid capture agents as disclosed in US Pat App. No. 13/554,623. These capture reagents were tested with patient samples for the ability to capture myeloma cells and compared their performance to anti-CD38/CD138 magnetic particle used in previous examples. The staining reagent used to detect myeloma cells contains anti-CD38 PE, anti-CD45 APC and anti-CD19 APC as described in U.S. Patent No. 9,618,515.

A total of 24 multiple myeloma patient samples were tested for CMMC enumeration using anti CD38/CD138, anti CS1/CD138 and anti BCMA/CD138 magnetic particles. The blood samples from multiple myeloma patients were collected in CellSave tubes and shipped to Janssen R&D, Huntingdon Valley by Conversant for the next day delivery. 4 ml of blood was used per test. The samples were processed within 24 hours on the CellTracks® AutoPrep® and then analyzed on the Cell Tracks Analyzer II® to enumerate CMMCs.

The results from patient samples are summarized in Tables 1 and 2. There is no significant difference in the

CMMC numbers between anti CD38/CD138 and anti BCMA/CD138 capture reagents (p value from TTEST = 0.4957) or between anti CD38/CD138 and anti CS1/CD138 capture reagents (p value from TEST = 0.4920). In addition, there is no significant difference in the number of samples positive at various levels of CMMCs between different capture reagents. This data demonstrates that antibodies to CS1 and BCMA can be used to capture multiple myeloma cells from blood.

**Table 1**: Number of CMMCs captured by different capture reagents

| | # of CMMCs/4.0ml Blood | | |
|---|---|---|---|
| Patient ID | CD38/CD138 FF | BCMA/CD138 FF | CS1/CD138 FF |
| 110035130 | 6 | 10 | 8 |
| 120078965 | 3 | 1 | 3 |
| 120079067 | 0 | 4 | 5 |
| 120039885 | 2230 | 2332 | 3102 |
| 01F2DBA50 | 280 | 455 | 664 |
| 110035126 | 12 | 18 | 36 |
| 1200816450 | 4 | 0 | 0 |
| 120081648 | 6 | 3 | 13 |
| 120039885 | 1429 | 2361 | 2775 |
| 120084708 | 45 | 25 | 39 |
| 120083239 | 4 | 11 | 8 |
| 100001092 | 1 | 3 | 1 |
| 120082176 | 0 | 0 | 2 |
| 110029686 | 6 | 10 | 17 |
| 120082966 | 1173 | 1147 | 1719 |
| 120081030 | 6818 | 3151 | 7397 |
| 110029076 | 717 | 1212 | 1134 |
| 110029078 | 81 | 96 | 116 |
| 120087278 | 584 | 667 | 720 |
| 120087409 | 0 | 1 | 0 |
| 120087493 | 23104 | 24600 | 19364 |
| 120081029 | 0 | 0 | 1 |
| 120087865 | 89 | 125 | 104 |
| 0019870DC | 14 | 6 | 6 |
| p value from TTEST | | 0.4957 | 0.4920 |

**Table** 2: Summary of percentage of patient samples positive at various levels of CMMCs with different capture reagents.

| | GD38/CD138 FF | BCMA/CD138 FF | CS1/CD138 FF |
|---|---|---|---|
| n | 24 | 24 | 24 |
| # of Samples ≥ 1 CMMCs | 20 (83%) | 21 (87%) | 22 (92%) |
| # of Samples ≥ 3 CMMCs | 19 (79%) | 19 (79%) | 19 (79%) |
| # of Samples ≥ 5 CMMCs | 16 (67%) | 16 (67%) | 18 (75%) |
| # of Samples ≥ 10 CMMCs | 13 (54%) | 15 (62%) | 14 (58%) |
| # of Samples ≥ 50 CMMCs | 10 (42%) | 10 (42%) | 10 (42%) |
| # of Samples ≥ 1000 CMMCs | 5 (21%) | 6 (25%) | 6 (25%) |

**EXAMPLE 2-CD138 Stabilization**

While CD138 (Syndican-1) magnetic particles alone can be used to capture myeloma cells as described in US Pat. No. 9,618,515 and as part of commercial kits previously described in this PCT application, the CD138 antigen has been shown to shed from the surface of myeloma cells. Glyoxal, an organic dialdehyde, which has some cell fixative properties was added to the CellSave blood collection tube (Menarini Silicon Biosystems, Huntingdon Valley, PA) in order to monitor the ability to stabilize the CD138 antigen for capture in both multiple myeloma cell lines, multiple myeloma patient samples, and age matched normal healthy donors. Using the methods described for creating anti CD 138 and anti CD138/38 ferrofluid capture agents as disclosed in US Pat. No. 9,618,515, these capture reagents were tested with cell lines, patient blood, and normal healthy donor blood samples collected in CellSave blood collection tubes containing glyoxal for the ability to capture myeloma cells. The staining reagent used to detect myeloma cells contains anti-CD38 PE, anti-CD45 APC, and anti-CD19 APC as described in U.S. Pat. No. 9,618,515.

The multiple myeloma cell line H929 was harvested from culture and placed into collection tubes containing either CellSave, CellSave plus glyoxal, or CellSecure, a proprietary preservative solution which also contains glyoxal. The cells were stored in the respective preservative solutions for 24, 48, 72 and 96 hours. At each time point, including a zero-time point just after harvest, cells were processed on the CELLTRACKS® AUTOPREP® and then analyzed on the CELLTRACKS ANALYZER II® to enumerate CMMC using CD138 ferrofluid capture reagent as described in U.S. Pat. No. 9,618,515. Total recovered cells at the zero-time point was compared to each of the subsequent time points. The results demonstrate that recovery of H929 cells stored in CellSave dropped at all time points after the zero-time point but did not decline when the cells were stored in either CellSave plus glyoxal or CellSecure suggesting that the CD138 antigen was preserved in the presence of glyoxal for at least 96 hours.

A total of 20 multiple myeloma patient samples and 10 normal healthy donor samples were tested for CMMC enumeration using anti CD138 and anti CD138/CD38 magnetic particles. The blood samples were collected in CellSave and CellSave plus glyoxal tubes and shipped to Menarini Silicon Biosystems R&D, Huntingdon Valley by Conversant for the next day delivery. 4ml of blood was used per test. Samples (4mL) were then processed on the CellSearch platform using the combination of CD138/CD38 and CD138 alone capture ferrofluid. The remaining blood was allowed to sit at room temperature. Of the 20 patient samples tested, 12 patients had measurable circulating multiple myeloma cells (CMMC). An additional 4mL blood sample from each of these patients was processed after 96 hours. In 12 of 12 (at 24 hours) and 11 of 12 (at 96 hours) of the patient samples with measurable CMMC, the number of CMMC detected in blood drawn into tubes containing CellSave + glyoxal using either CD138/CD38 ferrofluid or CD138 ferrofluid was greater than or equal to the number of CMMC detected using CD138/CD38 ferrofluid or CD138 ferrofluid from CellSave blood alone. This data strongly suggests that CD138 ferrofluid can be reliably used to capture CMMC from multiple myeloma patient blood drawn into tubes containing CellSave + glyoxal up to 96 hours post blood draw. An additional benefit to using CD138 is a dramatic decrease in the number of carry-over white blood cells in the assay making analysis much easier for the operator.

**Table** 3: Numbers of CMMCs collected from multiple myeloma patient blood samples by blood collection tube (CellSave or CellSave + Glyoxal), capture ferrofluid (CD138/38 or CD138 alone), and time (24 or 96 hours after blood draw).

| | CellSave | | | | | | CellSave + Glyoxal | | | | |
|---|---|---|---|---|---|---|---|---|---|---|---|
| Sample | CD138/38 (24) | CD138/38 (96) | | CD138 (24) | CD138 (96) | | CD138/38 (24) | CD138/38 (96) | | CD138 (24) | CD138 (96) |
| 201 | 1 | 1 | | 1 | 1 | | 12 | 11 | | 13 | 8 |
| 204 | 13 | 10 | | 17 | 9 | | 51 | 65 | | 240 | 169 |
| 205 | 10 | 9 | | 6 | 5 | | 14 | 20 | | 13 | 10 |
| 207 | 8 | 8 | | 7 | 5 | | 13 | 9 | | 7 | 7 |
| 212 | 437 | 366 | | 382 | 238 | | 600 | 613 | | 503 | 636 |
| 213 | 852 | 984 | | 1020 | 901 | | 1186 | 849 | | 1611 | 530 |
| 214 | 48 | 50 | | 16 | 18 | | 36 | 26 | | 45 | 32 |
| 215 | 4 | 2 | | 4 | 6 | | 9 | 10 | | 9 | 16 |
| 216 | 3605 | 3289 | | 2541 | 1961 | | 4452 | 5017 | | 5462 | 5756 |
| 217 | 4 | 5 | | 2 | 2 | | 4 | 5 | | 4 | 3 |
| 219 | 6 | 6 | | 0 | 3 | | 6 | 5 | | 6 | 6 |

(continued)

| | CellSave | | | | | | CellSave + Glyoxal | | | | |
|---|---|---|---|---|---|---|---|---|---|---|---|
| Sample | CD138/38 (24) | CD138/38 (96) | | CD138 (24) | CD138 (96) | | CD138/38 (24) | CD138/38 (96) | | CD138 (24) | CD138 (96) |
| 221 | 16 | 17 | | 4 | 9 | | 16 | 19 | | 13 | 25 |

**Table** 4: Number of unassigned events (e.g., white blood cells) detected in multiple myeloma patient blood samples by blood collection tube (CellSave or CellSave + Glyoxal), capture ferrofluid (CD138/38 or CD138 alone), and time (24 or 96 hours after blood draw).

| | CellSave | | | | | | CellSave + Glyoxal | | | | |
|---|---|---|---|---|---|---|---|---|---|---|---|
| Sample | CD138/38 (24) | CD138/38 (96) | | CD138 (24) | CD138 (96) | | CD138/38 (24) | CD138/38 (96) | | CD138 (24) | CD138 (96) |
| 201 | 22768 | 11659 | | 966 | 1024 | | 13266 | 4124 | | 2954 | 1797 |
| 204 | 30961 | 29834 | | 22357 | 21852 | | 40921 | 24971 | | 6541 | 3380 |
| 205 | 12765 | 9032 | | 2913 | 5709 | | 11796 | 3801 | | 1107 | 647 |
| 207 | 31658 | 29705 | | 6427 | 29379 | | 26951 | 12452 | | 2474 | 3182 |
| 212 | 21383 | 11568 | | 1940 | 6082 | | 21905 | 7953 | | 1543 | 988 |
| 213 | 19447 | 8898 | | 19144 | 8216 | | 8877 | 1498 | | 367 | 154 |
| 214 | 41346 | 31097 | | 8681 | 6199 | | 15803 | 9742 | | 3954 | 4286 |
| 215 | 10091 | 13622 | | 13423 | 29737 | | 7589 | 4407 | | 530 | 378 |
| 216 | 13052 | 10513 | | 24181 | 22937 | | 1894 | 2718 | | 1457 | 1236 |
| 217 | 24320 | 29323 | | 8582 | 13408 | | 14873 | 12417 | | 682 | 619 |
| 219 | 37111 | 28914 | | 3482 | 3584 | | 42226 | 32817 | | 1998 | 1530 |
| 221 | 14877 | 10605 | | 903 | 2162 | | 10743 | 11797 | | 435 | 404 |
| | | | | | | | | | | | |
| Mean | 23315 | 18731 | | 9417 | 12524 | | 18070 | 10725 | | 2004 | 1550 |

**Claims**

1. A reagent for capturing circulating multiple myeloma cells (CMMCs) from biological samples, comprising colloidal magnetic particles, wherein each of the colloidal magnetic particles is conjugated to both:

    (i) a first ligand that specifically binds to CD 138; and
    (ii) a second ligand selected from the group consisting of a ligand that specifically binds to CD2 subset-1 protein (CS1) and a ligand that specifically binds to B-cell maturation antigen (BCMA).

2. The reagent of claim 1, wherein the first ligand is selected from an antibody, an antibody fragment, a protein, a polypeptide, an enzyme, and an aptamer, preferably wherein the first ligand is an antibody, or fragment thereof; and/or wherein the second ligand is selected from an antibody, an antibody fragment, a protein, a polypeptide, an enzyme, and an aptamer, preferably wherein the second ligand is an antibody, or fragment thereof.

3. The reagent of any of claims 1-2, wherein the second ligand specifically binds to BCMA, wherein the second ligand specifically binds to CS1, or wherein said magnetic particles comprise a ligand that specifically binds to CS1 and a ligand that specifically binds to BCMA.

4. A composition comprising the reagent of any of claims 1-3 and a stabilizing agent, wherein said stabilizing agent is a dialdehyde, optionally wherein the dialdehyde is selected from the group consisting of glutaraldehyde, glyoxal, and combinations thereof.

**5.** The composition of claim 4, wherein the composition comprises CellSave® liquid or CellSecure® liquid.

**6.** The composition of any of claims 4-5, wherein the stabilizing agent is present in an amount of about 0.1 to about 50% w/v, preferably in an amount of about 0.3 to about 30% w/v, and more preferably in an amount of about 0.3 to about 5% w/v.

**7.** A kit for capturing rare cells from biological samples comprising

a) a reagent according to any one of claims 1-3 or a composition according to any one of claims 4-6; and
b) at least one additional marker selected from the group consisting of DAPI, and a ligand that specifically binds to a protein selected from: CS1, BCMA, CD19, CD45, CD 56, immunoglobulin lambda, immunoglobulin kappa, CD 200, and Ki67.

**8.** The kit of claim 7, wherein the kit further comprises a second additional marker and a third additional marker, optionally wherein the at least one additional marker is a ligand that specifically binds to CS1, the second additional marker is DAPI and the third additional marker is a ligand that specifically binds to CD45; and/or

the kit further comprising three additional markers, wherein at least one additional marker is a ligand that specifically binds to CS1, and the three additional markers are DAPI, a ligand that specifically binds to CD19, and a ligand that specifically binds to CD45; and/or
the kit further comprising four additional markers, wherein at least one additional marker is a ligand that specifically binds to CS1, and the four additional markers are a ligand that specifically binds to BCMA, DAPI, a ligand that specifically binds to CD19, and a ligand that specifically binds to CD45; and/or
wherein all additional markers that are not DAPI are antibodies or fragments thereof.

**9.** A method for capturing CMMCs from a biological sample obtained from a patient, comprising

a) contacting the biological sample with a reagent according to any one of claims 1-3 or a composition according to any one of claims 4-6; and
b) subjecting the sample of step (a) to a magnetic field to produce a separated fraction of magnetic particle-bound rare cells.

**10.** A method of detecting the amount of CMMCs in a biological sample from a patient, comprising

(a) contacting a biological sample obtained from a patient with a reagent according to any of claims 1-3 or a composition according any of claims 4-6;
(b) subjecting the sample of step (a) to a magnetic field to produce a separated fraction of magnetic particle-bound rare cells; and
(c) detecting the number of magnetic particle-bound rare cells.

**11.** The method of any of claims 9-10, further comprising contacting or treating the sample of step (a) with at least one additional marker selected from the group consisting of DAPI, and a ligand that specifically binds to a protein selected from: CS1, BCMA, CD19, CD45, CD 56, immunoglobulin lambda, immunoglobulin kappa, CD 200, and Ki67;

optionally further comprising contacting the sample with a second additional marker and a third additional marker, preferably wherein the at least one additional marker is a ligand that specifically binds to CS1, the second additional marker is DAPI and the third additional marker is a ligand that specifically binds to CD45; and/or
further comprising contacting the sample with three additional markers, wherein at least one additional marker is a ligand that specifically binds to CS1, and the three additional markers are DAPI, a ligand that specifically binds to CD19, and a ligand that specifically binds to CD45; and/or
further comprising contacting the sample with four additional markers, wherein at least one additional marker is a ligand that specifically binds to CS1, and the four additional markers are a ligand that specifically binds to BCMA, DAPI, a ligand that specifically binds to CD19, and a ligand that specifically binds to CD45.

**12.** The method of any of claims 9-11, wherein the sample has a volume of about 2 mL to about 10 mL, preferably of about 3 mL to about 7.5 mL, more preferably of about 4 mL or of about 7.5 mL; and/or wherein step (a) comprises contacting the biological sample with a composition according to any of claims 4-6.

13. A method of determining if a patient is a likely candidate for therapeutic intervention for a disease associated with abnormal plasma cells, comprising

(a) detecting the amount of CMMCs in a biological sample from a patient according to a method of any of claims 10-12; and
(b) determining if the number of CMMCs present in said sample, is equal to or greater than a normal range, wherein an amount of rare cells present in said sample greater than a normal range indicate the patient is a likely candidate for therapeutic intervention for a disease associated with abnormal plasma cells;

and/or

wherein the normal range of CMMCs in a patient sample is less than 7 CMMCs in about 2 mL to 10 mL of blood; and/or
wherein the disease associated with abnormal plasma cells is selected from multiple myeloma, monoclonal gammopathy of undetermined significance (MGUS), and smoldering multiple myeloma, preferably wherein the disease associated with abnormal plasma cells is multiple myeloma.

14. A method of determining whether a patient undergoing therapeutic intervention for a disease associated with abnormal plasma cells is being effectively treated, comprising

(a) detecting the amount of CMMCs in a biological sample from a patient according to a method of any of claims 10-12 at a first point in time; and
(b) detecting the amount of CMMCs in a biological sample from the patient according to a method of any of claims 10-12 at a second subsequent point in time; and
(c) comparing the numbers of CMMCs in a biological sample from the patient between the first point in time and the second subsequent point in time;
optionally wherein a lesser amount of CMMCs in the biological sample from patient at the second subsequent point in time indicates that the patient is being effectively treated;
and/or
wherein the disease associated with abnormal plasma cells is selected from multiple myeloma, monoclonal gammopathy of undetermined significance (MGUS), and smoldering multiple myeloma, preferably wherein the disease associated with abnormal plasma cells is multiple myeloma; and/or
wherein said therapeutic intervention is the administration of one of the following drugs daratumumab, isatux-imab, examethasone, cyclophosphamide, Vincristine, Bortezomib, Melphalan, Zometa, Aloxi, Lenalidomide,or Doxirubicin.

**Patentansprüche**

1. Reagenz zum Einfangen von zirkulierenden multiplen Myelomzellen ("circulating multiple myeloma cells", CMMCs) aus biologischen Proben, umfassend kolloidale magnetische Partikel, wobei jedes der kolloidalen magnetischen Partikel konjugiert ist mit beidem:

(i) einem ersten Liganden, der spezifisch an CD 138 bindet; und
(ii) einem zweiten Liganden, ausgewählt aus der Gruppe bestehend aus einem Liganden, der spezifisch an das CD2-Subset-1-Protein (CS1) bindet, und einem Liganden, der spezifisch an das B-Zell-Reifungsantigen ("B-cell maturation antigen", BCMA) bindet.

2. Reagenz nach Anspruch 1, wobei der erste Ligand aus einem Antikörper, einem Antikörperfragment, einem Protein, einem Polypeptid, einem Enzym und einem Aptamer ausgewählt ist, vorzugsweise wobei der erste Ligand ein Antikörper oder ein Fragment davon ist; und/oder wobei der zweite Ligand aus einem Antikörper, einem Anti-körperfragment, einem Protein, einem Polypeptid, einem Enzym und einem Aptamer ausgewählt ist, vorzugsweise wobei der zweite Ligand ein Antikörper oder ein Fragment davon ist.

3. Reagenz nach irgendeinem der Ansprüche 1-2, wobei der zweite Ligand spezifisch an BCMA bindet, wobei der zweite Ligand spezifisch an CS1 bindet oder wobei die magnetischen Partikel einen Liganden, der spezifisch an CS1 bindet, und einen Liganden, der spezifisch an BCMA bindet, umfassen.

**4.** Zusammensetzung, umfassend das Reagenz nach irgendeinem der Ansprüche 1-3 und ein Stabilisierungsmittel, wobei das Stabilisierungsmittel ein Dialdehyd ist, wobei das Dialdehyd optional aus der Gruppe ausgewählt ist, die aus Glutaraldehyd, Glyoxal und Kombinationen davon besteht.

**5.** Zusammensetzung nach Anspruch 4, wobei die Zusammensetzung CellSave®-Flüssigkeit oder CellSecure®-Flüssigkeit umfasst.

**6.** Zusammensetzung nach irgendeinem der Ansprüche 4-5, wobei das Stabilisierungsmittel in einer Menge von etwa 0,1 bis etwa 50 % w/v, vorzugsweise in einer Menge von etwa 0,3 bis etwa 30 % w/v und noch bevorzugter in einer Menge von etwa 0,3 bis etwa 5 % w/v vorhanden ist.

**7.** Kit zum Einfangen seltener Zellen aus biologischen Proben, umfassend

a) ein Reagenz gemäß irgendeinem der Ansprüche 1 bis 3 oder eine Zusammensetzung gemäß irgendeinem der Ansprüche 4 bis 6; und
b) mindestens einen zusätzlichen Marker, ausgewählt aus der Gruppe bestehend aus DAPI und einem Liganden, der spezifisch an ein Protein bindet, ausgewählt aus: CS1, BCMA, CD19, CD45, CD 56, Immunglobulin Lambda, Immunglobulin Kappa, CD 200 und Ki67.

**8.** Kit nach Anspruch 7, wobei das Kit ferner einen zweiten zusätzlichen Marker und einen dritten zusätzlichen Marker umfasst, wobei optional der mindestens eine zusätzliche Marker ein Ligand ist, der spezifisch an CS1 bindet, der zweite zusätzliche Marker DAPI ist und der dritte zusätzliche Marker ein Ligand ist, der spezifisch an CD45 bindet; und/oder

das Kit ferner drei zusätzliche Marker umfasst, wobei mindestens ein zusätzlicher Marker ein Ligand ist, der spezifisch an CS1 bindet, und die drei zusätzlichen Marker DAPI, ein Ligand, der spezifisch an CD19 bindet, und ein Ligand, der spezifisch an CD45 bindet, sind; und/oder
das Kit ferner vier zusätzliche Marker umfasst, wobei mindestens ein zusätzlicher Marker ein Ligand ist, der spezifisch an CS1 bindet, und die vier zusätzlichen Marker ein Ligand sind, der spezifisch an BCMA bindet, DAPI, ein Ligand, der spezifisch an CD19 bindet, und ein Ligand, der spezifisch an CD45 bindet; und/oder wobei alle zusätzlichen Marker, die nicht DAPI sind, Antikörper oder Fragmente davon sind.

**9.** Verfahren zum Nachweis von CMMCs aus einer biologischen Probe, die einem Patienten gewonnen wurde, umfassend

a) Inkontaktbringen der biologischen Probe mit einem Reagenz gemäß irgendeinem der Ansprüche 1-3 oder einer Zusammensetzung gemäß irgendeinem der Ansprüche 4-6; und
b) Aussetzen der Probe aus Schritt (a) an ein Magnetfeld, um eine abgetrennte Fraktion von magnetischen Partikeln gebundenen seltenen Zellen zu erzeugen.

**10.** Verfahren zum Nachweis der Menge an CMMCs in einer biologischen Probe, die von einem Patienten gewonnen wurde, umfassend

(a)Inkontaktbringen einer von einem Patienten gewonnenen biologischen Probe mit einem Reagenz gemäß irgendeinem der Ansprüche 1-3 oder einer Zusammensetzung gemäß irgendeinem der Ansprüche 4-6;
(b) Aussetzen der Probe aus Schritt (a) an ein Magnetfeld, um eine getrennte Fraktion von seltenen Zellen, die an magnetische Partikel gebunden sind, zu erzeugen; und
(c) Detektieren der Anzahl der seltenen Zellen, die an magnetische Partikel gebunden sind.

**11.** Verfahren nach einem der Ansprüche 9-10, das ferner das Inkontaktbringen oder Behandeln der Probe aus Schritt (a) mit mindestens einem zusätzlichen Marker umfasst, der aus der Gruppe ausgewählt ist, die aus DAPI und einem Liganden besteht, der spezifisch an ein Protein bindet, ausgewählt aus: CS1, BCMA, CD19, CD45, CD 56, Immunglobulin Lambda, Immunglobulin Kappa, CD 200 und Ki67;

optional ferner umfassend das Inkontaktbringen der Probe mit einem zweiten zusätzlichen Marker und einem dritten zusätzlichen Marker, wobei vorzugsweise der mindestens eine zusätzliche Marker ein Ligand ist, der spezifisch an CS1 bindet, der zweite zusätzliche Marker DAPI ist und der dritte zusätzliche Marker ein Ligand ist, der spezifisch an CD45 bindet; und/oder

ferner umfassend das Inkontaktbringen der Probe mit drei zusätzlichen Markern, wobei mindestens ein zusätzlicher Marker ein Ligand ist, der spezifisch an CS1 bindet, und die drei zusätzlichen Marker DAPI, ein Ligand, der spezifisch an CD19 bindet, und ein Ligand, der spezifisch an CD45 bindet, sind; und/oder
ferner umfassend das Inkontaktbringen der Probe mit vier zusätzlichen Markern, wobei mindestens ein zusätzlicher Marker ein Ligand ist, der spezifisch an CS1 bindet, und die vier zusätzlichen Marker ein Ligand sind, der spezifisch an BCMA bindet, DAPI, ein Ligand, der spezifisch an CD19 bindet, und ein Ligand, der spezifisch an CD45 bindet.

**12.** Verfahren nach einem der Ansprüche 9-11, wobei die Probe ein Volumen von etwa 2 ml bis etwa 10 ml, vorzugsweise von etwa 3 ml bis etwa 7,5 ml, noch bevorzugter von etwa 4 ml oder von etwa 7,5 ml aufweist; und/oder wobei Schritt (a) das Inkontaktbringen der biologischen Probe mit einer Zusammensetzung gemäß einem der Ansprüche 4-6 umfasst.

**13.** Verfahren zum Bestimmen, ob ein Patient ein wahrscheinlicher Kandidat für eine therapeutische Intervention bei einer Erkrankung ist, die mit abnormalen Plasmazellen assoziiert ist, umfassend

(a) Detektieren der Menge an CMMCs in einer biologischen Probe eines Patienten gemäß einem Verfahren nach einem der Ansprüche 10-12; und
(b) Bestimmen, ob die Anzahl der in der Probe vorhandenen CMMCs gleich oder größer als ein Normalbereich ist, wobei eine Menge seltener Zellen, die in der Probe vorhanden sind und größer als ein Normalbereich ist, darauf hinweist, dass der Patient ein wahrscheinlicher Kandidat für eine therapeutische Intervention für eine Krankheit ist, die mit abnormalen Plasmazellen assoziiert ist;

und/oder

wobei der normale Bereich von CMMCs in einer Patientenprobe weniger als 7 CMMCs in etwa 2 ml bis 10 ml Blut beträgt; und/oder
wobei die Krankheit, die mit abnormalen Plasmazellen assoziiert ist, aus multiplem Myelom, monoklonaler Gammopathie unbestimmter Signifikanz (MGUS) und schwelgendem multiplem Myelom ausgewählt ist, vorzugsweise wobei die Krankheit, die mit abnormalen Plasmazellen assoziiert ist, multiples Myelom ist.

**14.** Verfahren zum Bestimmen, ob ein Patient, der sich einer therapeutischen Intervention für eine mit abnormalen Plasmazellen assoziierte Krankheit unterzieht, wirksam behandelt wird, umfassend

(a) Detektieren der Menge an CMMCs in einer biologischen Probe eines Patienten gemäß einem Verfahren nach einem der Ansprüche 10 bis 12 zu einem ersten Zeitpunkt; und
(b) Detektieren der Menge an CMMCs in einer biologischen Probe von dem Patienten gemäß einem Verfahren nach einem der Ansprüche 10 bis 12 zu einem zweiten, späteren Zeitpunkt; und
(c) Vergleichen der Anzahl an CMMCs in einer biologischen Probe von dem Patienten zwischen dem ersten Zeitpunkt und dem zweiten, späteren Zeitpunkt;
wobei optional eine geringere Menge an CMMCs in der biologischen Probe des Patienten an dem zweiten nachfolgenden Zeitpunkt anzeigt, dass der Patient wirksam behandelt wird; und/oder
wobei die Krankheit, die mit abnormalen Plasmazellen assoziiert ist, aus dem multiplen Myelom, der monoklonalen Gammopathie unbestimmter Signifikanz ("monoclonal gammopathy of undetermined significance", MGUS) und dem schwelenden multiplen Myelom ausgewählt ist, vorzugsweise wobei die Krankheit, die mit abnormalen Plasmazellen assoziiert ist, das multiple Myelom ist; und/oder
wobei die therapeutische Intervention die Verabreichung eines der folgenden Medikamente ist: Daratumumab, Isatuximab, Examethason, Cyclophosphamid, Vincristin, Bortezomib, Melphalan, Zometa, Aloxi, Lenalidomid oder Doxirubicin.

## Revendications

**1.** Réactif pour capturer des cellules de myélome multiple circulantes (CMMC) dans des échantillons biologiques , comprenant des particules magnétiques colloïdales, dans lequel chacune des particules magnétiques colloïdales est conjuguée à la fois à :

(i) un premier ligand se liant spécifiquement à CD 138 ; et

(ii) un deuxième ligand sélectionné dans le groupe consistant en un ligand se liant spécifiquement à la protéine CD2 subset-1 (CS1) et un ligand se liant spécifiquement à l'antigène de maturation des cellules B (BCMA).

**2.** Réactif selon la revendication 1, dans lequel le premier ligand est sélectionné parmi un anticorps, un fragment d'anticorps, une protéine, un polypeptide, une enzyme, et un aptamère, de préférence dans lequel le premier ligand est un anticorps, ou un fragment de celui-ci ; et/ou dans lequel le deuxième ligand est sélectionné parmi un anticorps, un fragment d'anticorps, une protéine, un polypeptide, une enzyme, et un aptamère, de préférence dans lequel le deuxième ligand est un anticorps, ou un fragment de celui-ci.

**3.** Réactif selon l'une quelconque des revendications 1 à 2, dans lequel le deuxième ligand se lie spécifiquement à BCMA, dans lequel le deuxième ligand se lie spécifiquement à CS1, ou dans lequel lesdites particules magnétiques comprennent un ligand se liant spécifiquement à CS1 et un ligand se liant spécifiquement à BCMA.

**4.** Composition comprenant le réactif selon l'une quelconque des revendications 1 à 3 et un agent stabilisant, dans laquelle ledit agent stabilisant est un dialdéhyde, facultativement dans laquelle le dialdéhyde est sélectionné dans le groupe consistant en glutaraldéhyde, glyoxal, et des combinaisons de ceux-ci.

**5.** Composition selon la revendication 4, dans laquelle la composition comprend du liquide CellSave® ou du liquide CellSecure®.

**6.** Composition selon l'une quelconque des revendications 4 à 5, dans laquelle l'agent stabilisant est présent en une quantité d'environ 0,1 % à environ 50 % p/v, de préférence en une quantité d'environ 0,3 % à environ 30 % p/v, et de manière davantage préférée en une quantité d'environ 0,3 % à environ 5 % p/v.

**7.** Kit pour capturer des cellules rares dans des échantillons biologiques comprenant,

a) un réactif selon l'une quelconque des revendications 1 à 3 ou une composition selon l'une quelconque des revendications 4 à 6 ; et
b) au moins un marqueur supplémentaire sélectionné dans le groupe consistant en le DAPI, et un ligand se liant spécifiquement à une protéine sélectionnée parmi : CS1, BCMA, CD19, CD45, CD 56, immunoglobuline lambda, immunoglobuline kappa, CD 200, et Ki67.

**8.** Kit selon la revendication 7, le kit comprenant en outre un deuxième marqueur supplémentaire et un troisième marqueur supplémentaire, facultativement dans lequel ledit au moins un marqueur supplémentaire est un ligand se liant spécifiquement à CS1, le deuxième marqueur supplémentaire est le DAPI et le troisième marqueur supplémentaire est un ligand se liant spécifiquement à CD45 ; et/ou

le kit comprenant en outre trois marqueurs supplémentaires, dans lequel au moins un marqueur supplémentaire est un ligand se liant spécifiquement à CS1, et les trois marqueurs supplémentaires sont le DAPI, un ligand se liant spécifiquement à CD19, et un ligand se liant spécifiquement à CD45 ; et/ou
le kit comprenant en outre quatre marqueurs supplémentaires, dans lequel au moins un marqueur supplémentaire est un ligand se liant spécifiquement à CS1, et les quatre marqueurs supplémentaires sont un ligand se liant spécifiquement à BCMA, au DAPI, un ligand se liant spécifiquement à CD19, et un ligand se liant spécifiquement à CD45 ; et/ou
dans lequel tous les marqueurs supplémentaires qui ne sont pas le DAPI sont des anticorps ou des fragments de ceux-ci.

**9.** Procédé pour capturer des CMMC dans un échantillon biologique obtenu d'un patient, comprenant

a) le fait de mettre en contact l'échantillon biologique avec un réactif selon l'une quelconque des revendications 1 à 3 ou une composition selon l'une quelconque des revendications 4 à 6 ; et
b) le fait de soumettre l'échantillon de l'étape (a) à un champ magnétique afin de produire une fraction séparée de cellules rares liées à des particules magnétiques.

**10.** Procédé pour détecter la quantité de CMMC dans un échantillon biologique provenant d'un patient, comprenant

(a) le fait de mettre en contact un échantillon biologique obtenu d'un patient avec un réactif selon l'une quelconque des revendications 1 à 3 ou une composition selon l'une quelconque des revendications 4 à 6 ;

(b) le fait de soumettre l'échantillon de l'étape (a) à un champ magnétique afin de produire une fraction séparée de cellules rares liées à des particules magnétiques ; et
(c) le fait de détecter le nombre de cellules rares liées à des particules magnétiques.

**11.** Procédé selon l'une quelconque des revendications 9 à 10, comprenant en outre le fait de mettre en contact ou de traiter l'échantillon de l'étape (a) avec au moins un marqueur supplémentaire sélectionné dans le groupe consistant en DAPI, et un ligand se liant spécifiquement à une protéine sélectionnée parmi : CS1, BCMA, CD19, CD45, CD 56, immunoglobuline lambda, immunoglobuline kappa, CD 200, et Ki67 ;

facultativement, comprenant en outre le fait de mettre en contact l'échantillon avec un deuxième marqueur supplémentaire et un troisième marqueur supplémentaire, de préférence dans lequel ledit au moins un marqueur supplémentaire est un ligand se liant spécifiquement à CS1, le deuxième marqueur supplémentaire est le DAPI et le troisième marqueur supplémentaire est un ligand se liant spécifiquement à CD45 ; et/ou
comprenant en outre le fait de mettre en contact l'échantillon avec trois marqueurs supplémentaires, dans lequel au moins un marqueur supplémentaire est un ligand se liant spécifiquement à CS1, et les trois marqueurs supplémentaires sont le DAPI, un ligand se liant spécifiquement à CD19, et un ligand se liant spécifiquement à CD45 ; et/ou
comprenant en outre le fait de mettre en contact l'échantillon avec quatre marqueurs supplémentaires, dans lequel au moins un marqueur supplémentaire est un ligand se liant spécifiquement à CS1, et les quatre marqueurs supplémentaires sont un ligand se liant spécifiquement à BCMA, au DAPI, un ligand se liant spécifiquement à CD19, et un ligand se liant spécifiquement à CD45.

**12.** Procédé selon l'une quelconque des revendications 9 à 11, dans lequel l'échantillon présente un volume d'environ 2 mL à environ 10 mL, de préférence d'environ 3 mL à environ 7,5 mL, de manière davantage préférée d'environ 4 mL ou d'environ 7,5 mL ; et/ou dans lequel l'étape (a) comprend le fait de mettre en contact l'échantillon biologique avec une composition selon l'une quelconque des revendications 4 à 6.

**13.** Procédé pour déterminer si un patient est un candidat probable pour une intervention thérapeutique pour une maladie associée à des plasmocytes anormaux, comprenant

(a) le fait de détecter la quantité de CMMC dans un échantillon biologique provenant d'un patient selon un procédé selon l'une quelconque des revendications 10 à 12 ; et,
(b) le fait de déterminer si le nombre de CMMC présents dans ledit échantillon, est égal à, ou est supérieur à une plage normale, dans lequel une quantité de cellules rares présentes dans ledit échantillon supérieure à une plage normale indique que le patient est un candidat probable pour une intervention thérapeutique pour une maladie associée à des plasmocytes anormaux ;

et/ou

dans lequel la plage normale de CMMC dans un échantillon de patient est inférieure à 7 CMMC dans environ 2 mL à 10 mL de sang ; et/ou
dans lequel la maladie associée à des plasmocytes anormaux est sélectionnée parmi le myélome multiple, la gammopathie monoclonale de signification indéterminée (MGUS), et le myélome multiple indolent, de préférence dans lequel la maladie associée à des plasmocytes anormaux est le myélome multiple.

**14.** Procédé pour déterminer si un patient subissant une intervention thérapeutique pour une maladie associée à des plasmocytes anormaux est traité de manière efficace, comprenant

(a) le fait de détecter la quantité de CMMC dans un échantillon biologique provenant d'un patient selon un procédé selon l'une quelconque des revendications 10 à 12 à un premier point dans le temps ; et,
(b) le fait de détecter la quantité de CMMC dans un échantillon biologique provenant du patient selon un procédé selon l'une quelconque des revendications 10 à 12 à un deuxième point dans le temps, subséquent ; et,
(c) le fait de comparer les nombres de CMMC dans un échantillon biologique provenant du patient entre le premier point dans le temps et le deuxième point dans le temps, subséquent ;
facultativement dans lequel une quantité inférieure de CMMC dans l'échantillon biologique provenant du patient au deuxième point dans le temps subséquent indique que le patient est traité de manière efficace ; et/ou dans lequel la maladie associée
à des plasmocytes anormaux est sélectionnée parmi le myélome multiple, la gammopathie monoclonale de

signification indéterminée (MGUS), et le myélome multiple indolent, de préférence dans lequel la maladie associée à des plasmocytes anormaux est le myélome multiple ; et/ou
dans lequel ladite intervention thérapeutique est l'administration de l'un des médicaments suivants daratumumab, isatuximab, dexaméthasone, cyclophosphamide, Vincristine, Bortézomib, Melphalan, Zometa, Aloxi, lénalidomide, ou doxorubicine.

FIGURE 1

H929 Cells Recovered Using CD138FF CMMC Kit
H929 Cells Recovered
250
200
150
100
50
0
0 Hour
24 Hour
48 Hour
72 Hour
96 Hour
CellSave
CellSecure
CellSave + Glyoxal
Blood Preservative

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9618515 B
- US 6365362 B
- US 5597531 A
- US 5698271 A
- US 7901950 B
- US 7011794 B
- US 554623


### Non-patent literature cited in the description


- **ANDREE et al.** Challenges in circulating tumor cell detection by the CellSearch system,. *Molecular Oncology*, 2016, vol. 10, 395-407
- **SWENNENHUIS et al.** Improving the CellSearch system. *Expert Review of Molecular Diagnostics*, 2016, vol. 16 (12), 1291-1305